# EUROPEAN PATENT APPLICATION

(11) **EP 4 306 138 A1**
(43) Date of publication of application: **17.01.2024**
(21) Application number: 22767284.7
(22) Date of filing: 11.03.2022
(51) Int. Cl.: A61L 15/28, A61K 31/715, A61K 47/36, A61P 7/04

(54) **HEMOSTATIC MATERIAL**

(30) Priority: 12.03.2021 JP 2021040247
(71) Applicant: Artisan Lab Co., Ltd., Tokyo 152-0035 (JP); Nipro Corporation, Osaka-shi, Osaka 531-8510 (JP)
(72) Inventor: SHIBATA, Kazuhiko, Tokyo 152-0035 (JP)
(74) Representative: McWilliams, David John
(86) International application number: PCT/JP2022/011077
(87) International publication number: WO 2022/191330

(57) **Abstract**

Provided is an inexpensive, highly effective hemostatic material that can be used safely for a larger number of people. Provided is a hemostatic material for stopping bleeding that contains a cationic polysaccharide represented by formula (I) below. In a cationic polysaccharide, at least one R¹ represents -R⁷-N⁺(R⁸)(R⁹)(R¹⁰)·X⁻; R² represents OR¹ or H; R³ represents H or OR¹; R⁴ represents a galactose derivative or H; R⁵ represents R¹ or R⁶; R⁷ in R¹ represents C₁₋₆ alkylene or C₂₋₆ hydroxy alkylene; R⁸, R⁹, and R¹⁰ represent the same or different C₁₋₆ alkyl, C₁₋₆ alkenyl, O-C₁₋₆ alkyl, or the like; and X⁻ may represent an anionic group.

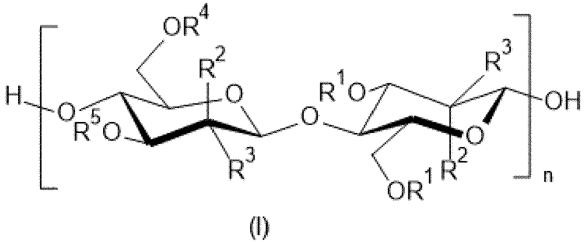

## Description

### [Technical Field]

The present invention relates to a hemostatic material.

### [Background Art]

Since ancient times, various hemostatic methods, such as compression, topical medication, cauterization, and ligation, have been tried to prevent death from massive bleeding in situations such as injuries due to war, hunting, and labor, and amputations. Until modern times, however, cauterization with a hot iron was a predominant method because ligation was a complicated procedure and a thread used for ligature caused suppuration, whereas cauterization was able to prevent suppuration.

The revival of ligation during the Renaissance and the invention of tourniquet in the 17th century, which enabled powerful temporary hemostasis, resulted in the widespread use of the ligation. Advances in hemostasis have been made with knowledge of vascular anatomy, elucidation of hemostatic mechanisms, improvement of forceps and ligatures, and invention of disinfection methods.

Compression hemostasis is commonly used by ordinary people and military personnel when they try to stop bleeding in an inadequately equipped area. A topical hemostatic material to assist this process is a material formed by fiberizing sodium calcium salt of alginic acid extracted from brown algae (Patent Literature 1). In recent years, chitosan extracted from outer shells of shellfish (Patent Literatures 2 and 3) have been adopted by the US Army and others as a strong hemostatic material.

### [Citation List]

### [Patent Literature]

[PTL 1] Japanese Translation of PCT International Application, Publication No. Hei 7-508277
[PTL 2] Japanese Translation of PCT International Application, Publication No. 2011-518592
[PTL 3] Japanese Unexamined Patent Application, Publication No. 2013-133287

### [Summary of Invention]

### [Technical Problem]

However, chitosan is likely to contain allergens that cause shellfish allergy. Therefore, those who are allergic to shellfish may experience severe allergic reactions when using a hemostatic material or bleeding dressing made from chitosan. In the case of using a preparation made from chitosan for bleeding points, the preparation should be prescribed with caution to people with shellfish allergy, and if possible, a different preparation should be used. However, there is a potential risk of developing an allergy upon prescription, since a person to whom the preparation is prescribed may not be aware in advance that he or she has a shellfish allergy. Moreover, a product made from chitosan is expensive and thus currently used only for severe bleeding. Since massive bleeding is a direct life-threatening event, there is a need for inexpensive raw materials that can stop bleeding faster.

The present invention has been made in view of such circumstances, and an object thereof is to provide an inexpensive, highly effective hemostatic material that can be used safely for a larger number of people.

### [Solution to Problem]

To solve the aforementioned problems, one aspect of the present invention is to provide a hemostatic material containing a cationic polysaccharide represented by formula (I) shown below.

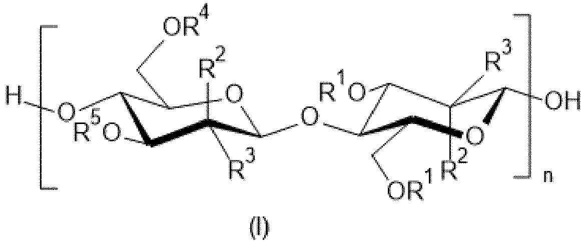

In the formula (I),
at least one R¹ represents -R⁷-N⁺(R⁸)(R⁹)(R¹⁰)·X⁻, and other R¹'s each represent -H,
R² represents OR¹ or H,
R³ represents H or OR¹,
R⁴ has a structure represented by formula (II) below or represents H,

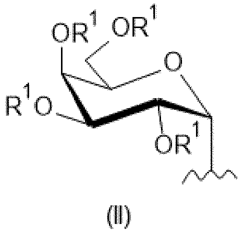

R⁵ represents R¹ or R⁶,
in which R⁶ has a structure represented by formula (III) below, 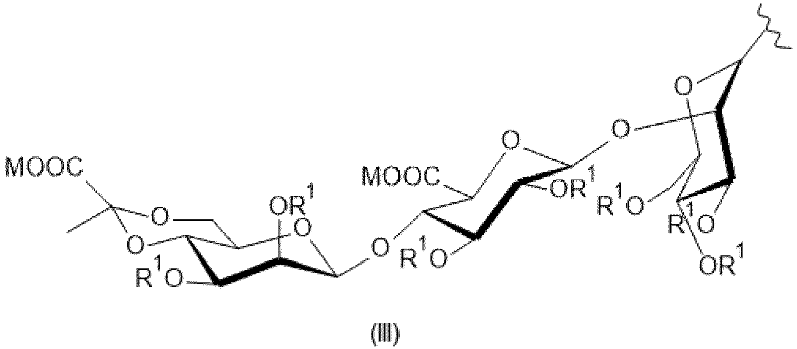
M represents Na, K, or 1/2 Ca,
in R¹, R⁷ represents C₁₋₆ alkylene or C₂₋₆ hydroxy alkylene, and R⁸, R⁹, and R¹⁰ represent the same or different C₁₋₆ alkyl, C₁₋₆ alkenyl, O-C₁₋₆ alkyl, and heteroalkyl or heteroalkenyl denoted by CₐY_{b}, where Y represents a heteroatom, a + b is 4 to 6, and in a case of a nitrogen atom being a binding site, a saturated or unsaturated five- or six-membered ring containing the nitrogen atom is formed, and
X- represents an anionic group.

In the above one aspect, the at least one R¹ in the formula (I) represents -CH₂CH(OH)CH₂-N⁺(R⁸)(R⁹)(R¹⁰)·X⁻, where R⁸, R⁹, and R¹⁰ each represent a methyl group or an ethyl group, and X⁻ may represent a halide ion.

In the above one aspect, the cationic polysaccharide represented by the formula (I) may be cationic guar gum.

In the above one aspect, the cationic guar gum may be guar hydroxypropyltrimonium chloride or hydroxypropyl guar hydroxypropyltrimonium chloride.

In the above one aspect, the cationic polysaccharide represented by the formula (I) may be cationic xanthan gum.

In the above one aspect, the cationic xanthan gum may be xanthan hydroxypropyltrimonium chloride.

### [Advantageous Effects of Invention]

The hemostatic material according to the present invention can be used safely for a larger number of people, is inexpensive, and has a higher hemostatic effect than general-purpose products.

### [Description of Embodiments]

An embodiment of the hemostatic material according to the present invention is described below.

In 2015, the present inventor of this application measured the time required for blood to clot after being mixed with cationic cellulose and found that cationic cellulose could clot blood in a shorter time, about one-fifth to one-sixth of that of chitosan, which is commonly used as a blood coagulant (Japanese Patent No. 6716841).

Cationic cellulose, which can clot blood at an early stage, has been commercialized as a hemostatic material and has been revealed to have excellent hemostatic ability (CATIONIC CELLULOSE BASED PAD STOPS BLEEDING WITHIN FIVE MINUTES AFTER HEMODIALYSIS, Kazuhiko Shibata, Hirokazu Morita, Mitsutaka Ueda, Shigeru Nakai, Nephrology Dialysis Transplantation, Volume 35, Issue Supplement 3, June 2020, gfaa 142. p.1364).

Hemostasis in a shorter time reduces the amount of blood loss. In some cases, the difference in the amount of blood loss may mean the difference between life and death. For this reason, the stronger the hemostatic material and the faster it clots blood, the more likely the material is to save lives. Recently, there has been a demand for a hemostatic material that can stop bleeding in a shorter time than cationic cellulose, and now the present inventor of this application has found that cationic guar gum and cationic xanthan gum each exhibit a higher hemostatic effect than cationic cellulose.

### Details are described below.

Guar gum is a water-soluble polysaccharide having a molecular weight of approximately 200,000 to 300,000 obtained from the seeds of guar (scientific name: Cyamopsis tetragonolobus), an annual legume. The guar gum is composed of mannose in the main chain and galactose in the side chain. The mannose and galactose have a hydroxy group and/or hydroxymethyl group. Cationic guar gum is a water-soluble cationic polymer. The cationic guar gum has a structure in which the aforementioned hydroxy group and/or hydroxymethyl are converted into a quaternary ammonium group. The cationic guar gum is utilized in various fields as a thickener and a gelling agent.

The following are some of the known characteristics of the cationic guar gum:
- When added to shampoo and conditioner, it improves feel, gloss, ease of styling and combing of hair.
- When added to skin care products, it has, for example, a skin softening effect, a water retention effect, a pH buffering effect, and a keratin damage suppression effect.

Xanthan gum is a polysaccharide having a molecular weight of about 2 million or 13 million to 15 million, obtained by fermentation of starches such as corn with the bacterium Xanthomonas campestris. The xanthan gum is composed of repeating unit structures consisting of two molecules of glucose in the main chain and two molecules of mannose and one molecule of glucuronic acid in the side chain. The cationic xanthan gum is utilized in various fields as a thickener and an emulsion stabilizer.

As the characteristics of the cationic xanthan gum, for example, when added to skin care and hair care products, it can improve the quality of foam during washing and emulsion stability.

The general formula (I) of the cationic polysaccharide contained in the hemostatic material according to the present embodiment is shown below.

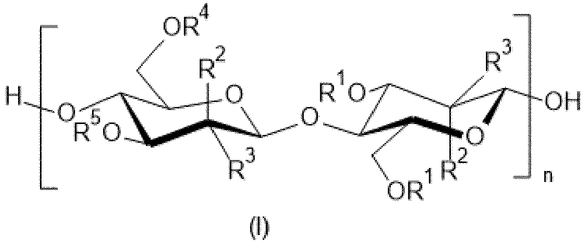

At least one R¹ in the formula (I) represents -R⁷-N⁺(R⁸)(R⁹)(R¹⁰)·X⁻, and other R¹'s each represent -H. R⁷ in the formula (I) represents C₁₋₆ alkylene or C₂₋₆ hydroxy alkylene. Examples of the C₁₋₆ alkylene include methylene, ethylene, propylene, isopropylene, butylene, isobutylene, tert-butylene, pentylene, and hexylene. Examples of the C₂₋₆ hydroxy alkylene include 1-hydroxyethylene, 2-hydroxyethylene, and 1-hydroxypropylene.

In the formula (I), R² represents OR¹ or H, R³ represents H or OR¹, and R⁴ has a structure represented by formula (II) below.

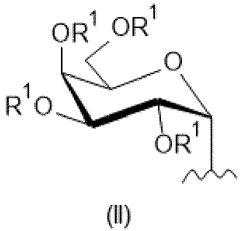

Specifically, when the cationic polysaccharide is cationic guar gum, in the formula (I), R² represents OR¹, R³ represents H, and R⁴ represents a galactose derivative and has a structure represented by the formula (II) above. When the cationic polysaccharide is cationic xanthan gum, in the formula (I), R² represents H, R³ represents OR¹, and R⁴ represents H.

In the formula (I), R⁵ represents R¹ or R⁶, and R⁶ has a structure represented by formula (III) below in which two molecules of a mannose derivative and one molecule of glucuronic acid are linked. In the formula (III), -COOM represents a carboxylate, where M is Na, K, or 1/2 Ca.

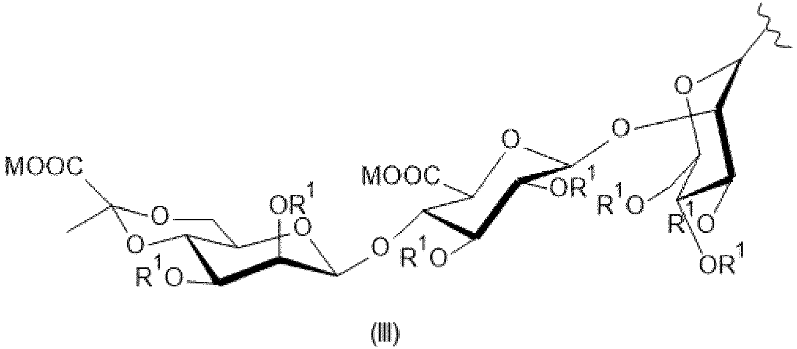

Specifically, when the cationic polysaccharide is cationic guar gum, R⁵ represents R¹. When the cationic polysaccharide is cationic xanthan gum, R⁵ represents R⁶, in which R⁶ has a structure represented by the formula (III) above.

In R¹ in the formula (I), R⁸, R⁹, and R¹⁰ represent the same or different C₁₋₆ alkyl, C₁₋₆ alkenyl, O-C₁₋₆ alkyl, and heteroalkyl or heteroalkenyl denoted by CₐY_{b}, where Y is a heteroatom. Examples of representative alkyl groups include a methyl group, an ethyl group, a propyl group, and an isopropyl group.

X⁻ in the formula (I) represents an anionic group and is paired with a quaternary ammonium cation -R⁷-N⁺(R⁸)(R⁹)(R¹⁰). Examples of the anionic group include a halide ion, such as F⁻, Cl⁻, Br-, and I-.

The cationic guar gum and cationic xanthan gum used in the present embodiment can be purchased as commercial products. In addition, the cationic guar gum can be synthesized through a known method of reacting guar gum with an organic ammonium salt (for example, Japanese Unexamined Patent Application, Publication No. Hei 9-176203). Furthermore, the cationic xanthan gum can be synthesized through a known method of reacting xanthan gum with an organic ammonium salt (for example, WO 90/06174). The cationic guar gum according to the present embodiment may be guar hydroxypropyltrimonium chloride or hydroxypropyl guar hydroxypropyltrimonium chloride. The cationic xanthan gum according to the present embodiment may be xanthan hydroxypropyltrimonium chloride.

### Experiment 1. Experiment to Confirm Hemostatic Property of Cationic Guar Gum and Cationic Xanthan Gum

The hemostatic properties of cationic guar gum and cationic xanthan gum according to the present embodiment were confirmed by comparing the clotting time of blood with that of a control substance using Helena C-ACT tubes (from Helena Laboratories Corporation), which are cartridges dedicated to a blood clotting measurement device. Since the C-ACT tubes contain Celite as a clotting activator, the Celite was used as a control substance in this experiment. Activated clotting time (ACT) was measured with Helena blood clotting analyzer Actalyke MINI or visually by several physicians.

First, the Celite was removed from the C-ACT tubes, and then the inside of the tubes was rinsed with distilled water. Subsequently, 15 mg each of JAGUAR C-17K (guar hydroxypropyltrimonium chloride, manufactured by Sansho Co., Ltd.) as cationic guar gum and Rhaball Gum^{®} CX (xanthan hydroxypropyltrimonium chloride, manufactured by DSP Gokyo Food & Chemical Co., Ltd.) as cationic xanthan gum were weighed with a precision scale and placed into the C-ACT tubes rinsed after the removal of the Celite. A butterfly needle was put into the elbow of a subject (inventor in this case) using a 22G injection needle and a 2.5 cc syringe to collect whole blood. Immediately after blood collection, 2.0 mL of collected blood was injected into each tube. C-ACT tubes containing Celite as a control substance were used as a control. In addition, the same experiment was conducted using POIZ C-150L (manufactured by Kao Corporation) as cationic cellulose. After the blood and a blood clotting accelerant (cationic guar gum, cationic xanthan gum, cationic cellulose, or Celite) were thoroughly mixed, the respective tubes were loaded into the main unit of the measurement device to measure the activated clotting time. The measurement results are shown in Table 1. According to the package insert, the normal ACT with C-ACT tubes is 105 to 130 seconds.

**[Table 1]**

| | | Control | Cationic polysaccharide | | |
|---|---|---|---|---|---|
| | | Celite | Cationic cellulose | Cationic xanthan gum | Cationic guar gum |
| Activated clotting time (sec) | Test 1 | 128 | 36 | 44 | 28 |
| | Test 2 | 118 | 64 | 66 | 20 |
| | Test 3 | 126 | 82 | 31 | 28 |
| | Test 4 | 124 | 71 | 80 | 32 |
| | Test 5 | 119 | 92 | 53 | 31 |
| Mean (sec) | | 123 | 69.0 | 54.8 | 27.8 |
| Standard deviation | | 4.3589 | 21.3073 | 19.0184 | 4.7117 |
| p-Value (t-test) | | | | 0.29854 | 0.00291 |

From the above results, it was confirmed that among the above-described cationic polysaccharides, the cationic guar gum most promoted clotting and could stop bleeding in a shorter time, about one-quarter to one-fifth of that of the Celite. It was also confirmed that the cationic xanthan gum could also clot in one-half or less the time required for clotting with Celite.

When cationic cellulose, which was previously found by the present inventor of this application as an effective substance for a hemostatic material, was used in the same experiment, the average time required for blood clotting was around 70 seconds. On the other hand, when the cationic guar gum of the present embodiment was used, the blood clotting time was shortened to a period more than 20 seconds but less than 30 seconds. As a result, it was confirmed that the cationic guar gum of the present embodiment could stop bleeding faster than cationic cellulose.

The present inventor of this application has previously found that the clotting time in the case of using cationic cellulose is only about one-fifth of the clotting time in the case of using chitosan, which is commonly used as a hemostatic material. Combined with the results of the present measurements, the cationic guar gum of the present embodiment shows a high potential to clot blood in one-tenth or less of the clotting time required when chitosan is used as a blood coagulant.

### Experiment 2. Experiment to Confirm Hemostatic Property of Cationic Guar Gum of Different Viscosity or Type

An experiment was conducted under the same conditions as in Experiment 1 described above using JAGUAR C-14S, JAGUAR C-17K, JAGUAR EXCEL, and JAGUAR C-162 (all manufactured by Sansho Co., Ltd.) as cationic guar gum to measure the activated clotting time. JAGUAR C-14S, JAGUAR C-17K, and JAGUAR EXCEL are each guar hydroxypropyltrimonium chloride and have different viscosities. JAGUAR C-162 is hydroxypropyl guar hydroxypropyltrimonium chloride and has a viscosity similar to that of JAGUAR EXCEL. The measurement results are shown in Table 2.

**[Table 2]**

| | | Control | Cationic guar gum | | | |
|---|---|---|---|---|---|---|
| | | Celite | EXCEL | C-17K | C-14S | C-162 |
| Activated clotting time (sec) | Test 1 | 120 | 25 | 21 | 24 | 20 |
| | Test 2 | 126 | 20 | 20 | 27 | 23 |
| Mean (sec) | | 123 | 23 | 21 | 26 | 22 |

From the above results, it was confirmed that all commercially available cationic guar gum, regardless of type and viscosity, could clot blood in about one-fifth the time compared to Celite.

### Experiment 3. Comparative Experiment of Hemostatic Property between Gauze Coated with Cationic Guar Gum and Commercially Available Hemostatic Gauze

An experiment was conducted to compare hemostatic properties of the cationic guar gum according to the present embodiment with those of commercially available hemostatic gauze and a control substance. JAGUAR C-17K and JAGUAR C-162 (both manufactured by Sansho Co., Ltd.) were used as cationic guar gum to prepare 1% or 2% aqueous solutions of each. Each of the aqueous solutions was applied to commercially available non-woven gauze and dried. Cationic cellulose (POIZ C-150L, manufactured by Kao Corporation) was used as one of the substances to be compared, and a 1% aqueous solution thereof was prepared and similarly applied to gauze and dried. As a control, commercially available gauze without any application was used. In addition, HemCon^{®} ChitoGauze^{®} (manufactured by Tricol Biomedical, Inc.) and CELOX RAPID (manufactured by Medtrade Products Limited), which are chitosan-coated non-woven gauzes, were used as commercial products for comparison of hemostatic properties.

Gauze coated with cationic guar gum or cationic cellulose was cut into 7 mm squares, and a plurality of sheets were stacked to a thickness of 3 mm and then compressed to prepare a test piece. The commercially available non-woven gauze, HemCon^{®} ChitoGauze^{®} and CELOX RAPID were also cut into 7 mm squares, and a plurality of sheets were stacked to a thickness of 3 mm and then compressed to prepare a test piece. Blood was collected from three healthy volunteers, and 100 µL of the obtained blood was added dropwise to each test piece to impregnate it. Every 2 minutes, a paper point (manufactured by DiaDent Group International) was brought into contact with each test piece, and the clotting time was determined when it was visually observed that blood no longer adhered to the contacted paper point. The results of Experiment 3 are shown in Table 3.

**[Table 3]**

| Conditions (type and concentration) | Clotting time (min) | | | Mean (min) |
|---|---|---|---|---|
| | Test 1 | Test 2 | Test 3 | |
| Only gauze (Control) | 21 | 30 | 35 | 28.7 |
| Cationic guar gum C-17K 1% | 7 | 17 | 15 | 13.0 |
| Cationic guar gum C-17K 2% | 7 | 7 | 11 | 8.3 |
| Cationic guar gum C-162 1% | 7 | 11 | 13 | 10.3 |
| Cationic guar gum C-162 2% | 11 | 11 | 11 | 11.0 |
| Cationic cellulose | 13 | 9 | 17 | 13.0 |
| CELOX RAPID | 7 | 11 | 11 | 9.7 |
| HemCon ChitoGauze | 9 | 11 | 11 | 10.3 |

The shorter the clotting time, the more hemostatic the test piece. The test pieces of the cationic guar gum used in the present experiment all had clotting times similar to those of commercially available hemostatic gauze. In particular, among the group of the test pieces tested in this experiment, it was found that gauze coated with a 2% aqueous solution of JAGUAR C-17K of cationic guar gum, exhibited the highest procoagulant effect, that is, the highest hemostatic property. HemCon^{®} ChitoGauze^{®} and CELOX RAPID are both hemostatic gauze with a history of adoption in the military and recognized as having very high hemostatic properties on a global scale. Compared with such highly hemostatic, commercially available hemostatic gauze, the gauze coated with a 2% aqueous solution of JAGUAR C-17K of cationic guar gum and the gauze coated with a 1% or 2% aqueous solution of JAGUAR C-162 were found to have hemostatic properties similar to or higher than those of the commercially available hemostatic gauze.

The hemostatic material according to the present invention contains cationic guar gum or cationic xanthan gum, which are contained in cosmetics, shampoo, and conditioner as substances safe even in direct contact with the skin. Thus, the hemostatic material according to the present invention can be used safely for a larger number of people and can protect the skin more effectively than cationic cellulose.

### (Hemostatic Function)

It is indisputable that a bleeding site must be stopped immediately after injury. Currently, the first choice of bleeding dressings for use in the case of bleeding at the bleeding site is KALTOSTAT^{®}, which contains an alginate, or a dressing containing chitosan. However, the alginate has a weaker hemostatic function than chitosan (Syota Suzuki, Kazuhiko Shibata, Randomized Trial comparing New Chitosan-Based Bandage with Kaltostat Hemostatic Dressing to Control Bleeding from Hemodialysis Puncture Site, Nephrol. Dial. Transplant., 2013, 28 (suppl.1), i226-i239.).

HemCon^{®} ChitoGauze^{®} and CELOX RAPID used in Experiment 3, when applied to the bleeding site, are known to rapidly plug the bleeding site and stop bleeding because chitosan attracts red blood cells and platelets in the blood. Such a function may be attributed to the fact that chitosan is positively charged under some conditions. Cationic cellulose is always positively charged independent of the pH of the surrounding environment. For this reason, the cationic cellulose exhibits better hemostatic properties than chitosan. Therefore, the cationic cellulose is useful as a component in a dressing used in the early stage of injury. On the other hand, no effective hemostatic effect could be confirmed at all in the case of using cationic paramylon, which is a sugar chain with likewise positively charged side chains. The present inventor of this application conducted confirmatory experiments on various compounds and found that the cationic guar gum and cationic xanthan gum according to the present invention could stop bleeding at a speed superior to that of the cationic cellulose. In cases such as life-threatening massive bleeding, shortening the time to achieve hemostasis is crucial to saving lives. Reduction in hemostasis time is a very valuable life-saving ability. From the results of Experiment 3, it is concluded that cationic guar gum in particular has better hemostatic ability than any commonly used hemostatic material.

Care should be taken in the use of commonly used chitosan-containing hemostatic materials in patients with shellfish allergy. In contrast, the cationic guar gum and cationic xanthan gum according to the present embodiment have no reported cases of severe skin sensitization symptoms to date, so it is presumed that there is little concern and more people can safely use them.

Next, aspects of the hemostatic material containing the cationic polysaccharide according to the present embodiment are described.

The hemostatic material in the present embodiment may be a gel cationic guar gum or cationic xanthan gum, allowing the hemostatic material containing cationic guar gum or cationic xanthan gum to be applied directly to the bleeding site. In this way, even if the bleeding site is irregularly shaped, the hemostatic material can be securely brought into contact with the bleeding site. Further, the bleeding can be stopped by covering the applied gel with gauze, film, or other material and applying pressure. After confirming hemostasis, the pressure can be released, and the gel can be continuously used as a bleeding dressing. The gel hemostatic material can be easily applied to bleeding sites with complicated shapes, such as cuts and deep bedsores.

The hemostatic material in the present embodiment can be made into a film containing cationic guar gum or cationic xanthan gum as the main component to stop bleeding, at least on the surface that may contact the bleeding site. Specifically, once the cationic guar gum or cationic xanthan gum is dissolved in a solvent such as water or alcohol to form a gel, the solvent is forced out of the gel, whereby the gel can be formed into a film. The film can be adjusted in thickness and density according to the intended use.

The film-shaped cationic guar gum may be formed from cationic guar gum alone or from a mixture of cationic guar gum and an additive such as an antimicrobial agent. The same method can be used to obtain film-shaped cationic xanthan gum.

The cationic guar gum or cationic xanthan gum can be made into a gel and contained in gauze, absorbent cotton, non-woven fabric, or urethane foam. The cationic guar gum or cationic xanthan gum can be formed into a film or prepared as a gel and applied to the bleeding site, and then a pad with water absorbency can be placed over it to absorb excess moisture and appropriately maintain a moist environment at the bleeding site as necessary for healing, even if a large amount of exudate is generated from the bleeding site.

The shape of the product as described above allows the user to easily apply the hemostatic material to the bleeding site. Cloth or non-woven fabrics containing or adsorbing the cationic guar gum or cationic xanthan gum can be used as hemostatic gauze.

There are cases where bleeding occurs with enormous amounts of exudate or does not stop easily. To deal with such bleeding, the hemostatic material may be powdered cationic guar gum or cationic xanthan gum. The cationic guar gum or cationic xanthan gum can be made into powder by adding an appropriate base or through spray drying, freeze drying, or other processes. If there is a large amount of bleeding or exudate from the bleeding site, the powder is sprinkled over the bleeding site, which is then covered with gauze, a waterproof film, or a water absorbent sheet. Hemostatic materials in such a form are easily used at an early stage of injury and can be applied with a high degree of freedom to bleeding sites of various shapes. Another form may be non-woven fabrics made from powdered cationic guar gum or cationic xanthan gum, which are kneaded with other materials. Such non-woven fabrics can function as self-adhesive non-woven fabrics, providing a larger contact area and absorbing exudates.

The present embodiment can provide a hemostatic material with a higher hemostatic action by using positively charged cationic guar gum or cationic xanthan gum.

## Claims

1. A hemostatic material for stopping bleeding, comprising a cationic polysaccharide represented by formula (I) below, 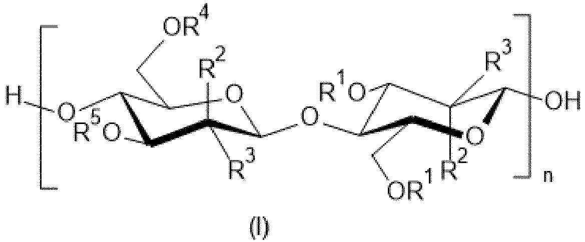 wherein
at least one R¹ represents -R⁷-N⁺(R⁸)(R⁹)(R¹⁰)·X⁻, and other R¹'s each represent -H,
R² represents OR¹ or H,
R³ represents H or OR¹,
R⁴ has a structure represented by formula (II) below or represents H, 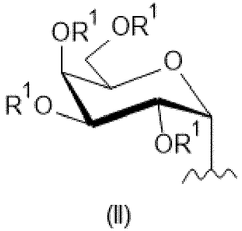
R⁵ represents R¹ or R⁶,
wherein R⁶ has a structure represented by formula (III) below, 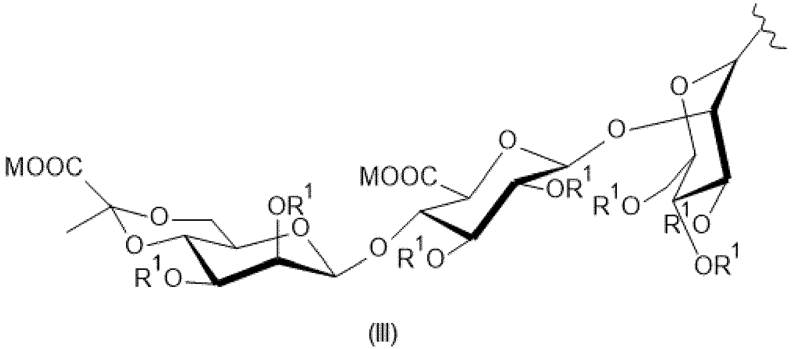
M represents Na, K, or 1/2 Ca,
in R¹, R⁷ represents C₁₋₆ alkylene or C₂₋₆ hydroxy alkylene, and R⁸, R⁹, and R¹⁰ represent the same or different C₁₋₆ alkyl, C₁₋₆ alkenyl, O-C₁₋₆ alkyl, and heteroalkyl or heteroalkenyl denoted by CₐY_{b}, where Y represents a heteroatom, a + b is 4 to 6, and in a case of a nitrogen atom being a binding site, a saturated or unsaturated five- or six-membered ring containing the nitrogen atom is formed, and
X- represents an anionic group.

2. The hemostatic material according to claim 1, wherein the at least one R¹ in the formula (I) represents -CH₂CH(OH)CH₂-N⁺(R⁸)(R⁹)(R¹⁰)·X⁻, where R⁸, R⁹, and R¹⁰ each represent a methyl group or an ethyl group, and X- represents a halide ion.

3. The hemostatic material according to claim 1, wherein the cationic polysaccharide represented by the formula (I) is cationic guar gum.

4. The hemostatic material according to claim 3, wherein the cationic guar gum is guar hydroxypropyltrimonium chloride or hydroxypropyl guar hydroxypropyltrimonium chloride.

5. The hemostatic material according to claim 1, wherein the cationic polysaccharide represented by the formula (I) is cationic xanthan gum.

6. The hemostatic material according to claim 5, wherein the cationic xanthan gum is xanthan hydroxypropyltrimonium chloride.
